# EUROPÄISCHE PATENTSCHRIFT

(11) **EP 3 554 421 B1**
(45) Veröffentlichungstag und Bekanntmachung des Hinweises auf die Patenterteilung: **02.12.2020**
(21) Anmeldenummer: 17818517.9
(22) Anmeldetag: 14.12.2017
(51) Int. Cl.: A61F 2/00, A61B 17/06

(54) **IMPLANTAT**
IMPLANT
IMPLANT

(30) Priorität: 15.12.2016 DE 202016107015 U
(43) Veröffentlichungstag der Anmeldung: 23.10.2019
(73) Patentinhaber: Reinmüller, Johannes, 65193 Wiesbaden (DE)
(72) Erfinder: Reinmüller, Johannes, 65193 Wiesbaden (DE)
(74) Vertreter: Weickmann & Weickmann PartmbB
(86) Internationale Anmeldenummer: PCT/EP2017/082787
(87) Internationale Veröffentlichungsnummer: WO 2018/109073

(56) Entgegenhaltungen:
- WO-A1-2007/111407
- WO-A1-2014/165221
- WO-A1-2015/072797
- FR-A1- 2 926 452
- GB-A- 2 492 900
- US-A1- 2002 019 670
- US-A1- 2015 327 988

## Beschreibung

Die Erfindung betrifft Kunststoff-Implantate zur Anwendung in der Medizin oder Veterinärmedizin.

In der ästhetischen Chirurgie sind verschiedene minimal invasive Verfahren entwickelt worden, um körperliche Fehlbildungen, Unfallfolgen oder Folgen anderer medizinischer Eingriffe sowie altersbedingte Veränderungen des Körpers zu behandeln. Zum Beispiel können plastisch-chirurgische Veränderungen oder Rekonstruktionen der Lippe durch Aufpolstern erfolgen, wobei biologische Polstermaterialien wie Körperfett, Bindegewebe, Hyaluronsäure, etc. oder Kunststoffpartikel verwendet werden. Solche Polstermaterialien werden üblicherweise durch kleine Schnitte im Lippenrot oder mit einer Spritze eingebracht. Durch Einbringen von fadenförmigen, unelastischen Implantaten gelingt es abgesunkene bzw. disloziere Weichteile zu reponieren und zu fixieren.

Biologische Polstermaterialien haben den Nachteil, dass sie vom Körper abgebaut werden und, im Falle von autologen Transplantaten zusätzliche Eingriffe an anderer Stelle zur Gewinnung des Transplantationsmaterials erfordern. Bisher verwendete Kunststoffpartikel als Polstermaterialien haben den Nachteil, dass sie Granulome hervorrufen und nicht ohne Weiteres wieder entfernt werden können. Kunststofffäden aus üblichen Nahtmaterialien sind unelastisch und führen durch Druckschädigung des Implantatlagers zu Resorption von gesundem Gewebe und zur nachfolgenden Dislokation bzw. Migration des Implantates.

WO 95/28894 beschreibt Implantate für medizinische Zwecke, die aus dünnen faltbaren Formkörpern mit einer für ein fluides Gleitmittel benetzbaren Oberfläche bestehen und die in Form von Folien, kapillaren

Röhren oder schlauchartigen Strängen ausgebildet sind.

US 2004/176856 offenbart ein Lippenimplantat aus Silikon mit einem konstanten Querschnitt von 2mm und einer Shore A Härte von 10.

US 2006/136070 A1 offenbart ein fadenförmiges Implantat aus Silikon mit einem Durchmesser von 0,1-1mm.

Ein Gegenstand der vorliegenden Erfindung ist ein Kunststoff-Implantat zur Anwendung in der Medizin oder Veterinärmedizin, das aus einem Strang eines im Wesentlichen nicht quellfähigen biologisch verträglichen Kunststoffmaterials in Form eines kompakten Zylinders mit einem Querschnitt von 0,6-1,8 mm besteht, dahingehend, dass es über die gesamte Länge einen einheitlichen kreisförmigen Querschnitt aufweist und das Kunststoffmaterial eine Shore A-Härte von 0-20 aufweist und aus einem Silikonkautschuk besteht.

Das Basismaterial des Implantats besteht aus einem kompakten und im Wesentlichen nicht quellfähigen Kunststoffmaterial, nämlich einem Silikonkautschuk wie etwa Polydimethylsiloxan. Das Material wird vorzugsweise so gewählt, dass es bei Kontakt mit Wasser bei 20°C eine Quellung von 10 Gew.-% oder weniger, insbesondere eine Quellung von 3 Gew.-% oder weniger, von 2 Gew.-% oder weniger oder von 1 Gew.-% oder weniger aufweist. Vorzugsweise ist das Basismaterial elastisch.

Das Implantat hat die Form eines massiven Zylinders, der einen kreisförmigen Querschnitt aufweist. Der Querschnitt zwischen 0,6 und 1,8 mm. Der Querschnitt ist über die gesamte Länge eines Implantats einheitlich.

In Beispielen, welche nicht unter die Erfindung fallen, kann das Implantat auch einen sich über die gesamte Länge verändernden alternierenden Querschnitt aufweisen (z.B. wie Bohnenschoten, Perlenkette, Rosenkranz), wobei auf beispielsweise 1 cm Länge des Zylinders ein kontinuierlich zu- bzw. abnehmender Querschnitt von z.B. 1,2-1,6 mm vorliegt. Die Oberfläche kann mit längs und/oder quer verlaufenden oder zirkulären Erhebungen und/oder Vertiefungen versehen sein. Vorzugsweise ist die Oberfläche glatt.

Die Härte des Implantats wird entsprechend den medizinischen Anforderungen bzw. den biologischen Geweben am Einsatzort ausgewählt.

Daraus ergeben sich Shore A-Härtegrade von 0-20 oder von 0-10, d.h. weiche Shore A-Härtegrade, wobei die Bestimmung des Shore A-Härtegrads bei 23°C entsprechend DIN ISO 7619-1 erfolgt.

Das fadenförmige Kunststoffimplantat besteht aus Silikonkautschuk und weist eine hohe Elastizität auf.

Die Länge des strangförmigen Implantats entspricht dem jeweiligen Einsatzzweck basierend auf der Anatomie des Einsatzortes. Üblicherweise beträgt die Länge mindestens 2 cm. Bei einer Anwendung im Lippenbereich, insbesondere am Lippenrot, kann die Länge etwa 4-6 cm, gegebenenfalls zzgl. einer Verlängerung für die Handhabung bei der Implantation, d.h. etwa 10 cm oder ein Vielfaches davon für Mehrfachanwendungen, entsprechen.

Weiterhin ist bevorzugt, dass das Implantat eine Oberflächenbeschichtung aufweist, z.B. eine Oberflächenbeschichtung zur Verbesserung der Gleitfähigkeit, eine Oberflächenbeschichtung zur Steigerung der Gewebeverträglichkeit und/oder eine Oberflächenbeschichtung zur Verhinderung von bakterieller Besiedlung.

Zur Verbesserung der Gleitfähigkeit kann die Oberfläche des Implantats mit einem hydrophilen, z.B. einem wässrigen, Gleitmittel oder einem hydrophoben Gleitmittel, z.B. einem Fett oder Öl, beschichtet sein. Alternativ kann die Gleitfähigkeit auch durch Verwendung von hydrophil oder hydrophob modifizierten Oberflächen hervorgerufen werden.

Als Beschichtung zur Steigerung der Gewebeverträglichkeit können an der Oberfläche des Implantats beispielsweise Polysaccharide, Glykosaminoglykane, Dextrane und dergleichen aufgebracht werden. Hyaluronsäure ist bevorzugt. Die Haftung solcher Beschichtungsmaterialien an der Implantatoberfläche kann verbessert werden, wenn diese zuvor mit einer Lösung oder Suspension eines Metallhydroxids vorbehandelt wurde, wie etwa in US 5,494,756 beschrieben ist.

Weiterhin kann das Implantat eine Oberflächenbeschichtung zur Verhinderung von mikrobieller, z.B. bakterieller Besiedlung aufweisen, wie etwa Antibiotika und/oder Silberpartikel enthalten.

Besonders bevorzugt enthält das Implantat eine dreifache Oberflächenbeschichtung, umfassend eine Oberflächenbeschichtung zur Steigerung der Gewebeverträglichkeit, eine Oberflächenbeschichtung zur Verbesserung der Gleitfähigkeit und eine Beschichtung zur Verhinderung von bakterieller Besiedlung. Besonders bevorzugt ist eine äußere Beschichtung zur Steigerung der Gewebeverträglichkeit, eine mittlere Beschichtung zur Verbesserung der Gleitfähigkeit und eine innere Beschichtung zur Verhinderung von bakterieller Besiedlung, d.h. direkt auf der Oberfläche des Implantats, vorgesehen.

In einer Ausführungsform der Erfindung enthält das Implantat im Inneren außer dem Kunststoffmaterial keine weiteren Bestandteile. In anderen Ausführungsformen kann das Material einen Farbstoff, ein Kontrastmittel, z.B. ein Röntgenkontrastmittel wie Bariumsulfat, einen pharmakologischen Wirkstoff wie etwa einen antimikrobiellen Wirkstoff, z.B. ein Antibiotikum, ein Antiseptikum, z.B. Taurolidin, und/oder einen biologischen Signalstoff enthalten. Alternativ oder zusätzlich kann das Implantat einen oder mehrere kontrastgebende Körper im Inneren, z.B. im Röntgenbild kontrastgebende Körper wie etwa Fäden, Streifen, Partikel und/oder Mikrosphären, enthalten.

Das erfindungsgemäße Implantat wird üblicherweise in sterilisierter und für medizinische Zwecke abgepackter Form bereitgestellt. Dabei kann das Implantat in Kombination mit einer oder mehreren Einführhilfen vorliegen, die ein Einbringen des Implantats in den Körper ermöglichen. Die Einführhilfe kann beispielsweise eine Trokarnadel, z.B. eine nicht schneidende Trokarnadel, gegebenenfalls zusammen mit einer äußeren Trokarhülse, umfassen. Dabei kann das Implantat mit einer Trokarnadel fest, z.B. atraumatisch, verbunden sein.

Die Trokarnadel und gegebenenfalls die Trokarhülse können aus chirurgischem Stahl und/oder aus einem für medizinische Zwecke geeigneten Kunststoffmaterial bestehen, wobei die Oberflächen gegebenenfalls mit einem Gleitmittel beschichtet sein oder eine gleitfähige Oberflächenbeschichtung, z.B. aus Teflon, aufweisen können. Die Trokarnadel ist bevorzugt aus einem Hartkunststoffmaterial ausgebildet und fest, z.B. atraumatisch, mit dem Kunststoff-Implantat verbunden.

Besonders bevorzugt besteht die Trokarnadel aus gehärtetem Silikonharz mit einer Länge von ca. 8-10 cm, die kontinuierlich in das weiche Implantat aus Silikonkautschuk übergeht. In dieser Ausführungsform entspricht der Durchmesser der Trokarnadel vorzugsweise dem Querschnitt des Implantats. Die Trokarnadel kann gerade oder gebogen sein, z.B. halbkreisförmig gebogen.

Das elastische, fadenförmige Kunsstoffimplantat kann auch zur Aufhängung bzw. Fixation von Weichteilen, z.B. im Gesicht, bevorzugt an den Wangen und Augenbrauen, eingesetzt werden. Hervorgehoben sei der Einsatz zur Behandlung von Gesichtslähmungen. Weiter Einsatzgebiete betreffen das Urogenitalsystem bei Mann und Frau, z.B. zur Behandlung von Inkontinenz bzw. zur elastischen Einengung von Hohlorganen. Hierzu kann es mit Einführhilfen in verschiedene gegbenenfalls verschiebliche Weichteilstrukturen bzw. Gewebsschichten eingebracht werden. Durch Verankerung an unelastischen, festen Gewebsstrukturen (Faszien, Knochen, Bändern) entsteht dadurch eine dynamische Aufhängung bzw. Zügelung. Auch eine ringförmige Anwendung ist Gegenstand des Schutzrechtes.

Bei einer nicht unter die Erfindung fallenden Ausführung des fadenförmigen, elastischen Implantates mit alternierenden Durchmessern in Form der Bohnenschote, der Perlenkette oder des Rosenkranzes können unterschiedliche Materialien Verwendung finden, so dass ein Komposit-Implantat entsteht. Entscheidend bleibt der zentrale elastische Faden, die Seele, bevorzugt aus Silikonkautschuk. Die daran symmetrisch oder asymmetrisch aufgereihten Formkörper können bestehen aus medizinisch verträglichen Metallen, Keramiken (Hydroxylapatit, Korallen) oder Kunststoffen (PMMA), resorbierbar oder nicht resorbierbar.

### Beispiele:

### Beispiel 1: Anwendung eines Implantats zum Aufbau des Lippenrots

Zunächst erfolgt eine lokale Betäubung der Oberlippe. Anschließend werden Stichinzisionen schleimhautseitig in den Mundwinkel vorgenommen. Anschließend wird ein nicht schneidender Trokar mit Nadel und Hülse, Innendurchmesser z.B. 1,6 mm, über die gesamte Länge der Oberlippe subkutan und/oder intramuskulär von rechts nach links oder umgekehrt eingebracht, so dass das stumpfe und das spitze Ende des Trokars jeweils das Gewebe der Oberlippe auf einer Seite überragen. Anschließend wird der Trokar aus der Hülse entfernt, die im Gewebe bleibt.

Ein Implantat, z.B. mit einer Länge von 10 cm und mit einem Querschnitt von 1,5 mm, wird in das Lumen der Hülse nach Entfernung der Trokarnadel eingeführt, bis das Implantat die Trokarhülse an beiden Enden überragt. Dann wird das Implantat an einem Ende gefasst bzw. fixiert und die Hülse aus dem Lippengewebe über das gegenüber liegende Ende entfernt. Das Implantat kann je nach Erfordernis gekürzt werden, so dass die Implantatenden innerhalb des Lippengewebes zu liegen kommen. Die Stichinzisionen werden z.B. mit einer Naht verschlossen.

Verschiedene Grade des Gewebeaufbaus lassen sich durch Variation des Implantatdurchmessers oder vorzugsweise durch Einbringen mehrerer parallel orientierter Implantate entweder jeweils in einem eigenen Gewebslager oder in einem gemeinsamen Gewebslager, z.B. durch Einziehen je eines Implantats sowohl an der Lippenrotgrenze als auch an der Lippenschleimhautobergrenze, der sogenannten Feuchtgrenze, einbringen.

In einer weiteren Ausführungsform kann die nicht schneidende Trokarnadel (ohne die äußere Hülse) mit dem Implantat atraumatisch verbunden sein. Dabei kann über die zuvor genannte Stichinzision die Trokarnadel in das Gewebe, z.B. der Lippe, eingeführt und an der gewünschten Austrittstelle aus dem Gewebe ausgeführt bzw. entnommen werden. Das fix verbundene Implantat folgt der Trokarnadel und kommt im Gewebe bestimmungsgemäß zu liegen. Die Trokarnadel kann dann vom Implantat, z.B. mit einer Schere, abgetrennt und das Implantat kann wie zuvor beschrieben auf die erforderliche Länge gekürzt werden. Dieser Vorgang kann mehrfach wiederholt werden.

### Beispiel 2: Anwendung eines Implantats zur Anhebung des (gelähmten) Mundwinkels

Über eine Stichinzision in der behaarten Schläfenregion wird mit einer Einführhilfe ein subkutaner Kanal präpariert und ein Silikonkautschuk-Faden subkutan in Richtung auf den Mundwinkel eingebracht. Dort kann er über eine weitere Stichinzision freigelegt und um 180 ° gewendet werden bzw. in einem neuen subkutanen Kanal, parallel zum ersten Kanal zur Schläfe zurückgeführt werden. An der unelastischen Faszie des Schläfenmuskels können die beiden Enden des elastischen Fadens unter adäquater Spannung fixiert werden, z.B. durch Verknotung. Die Stichinzisionen werden danach mit herkömmlichen Nähten verschlossen.

## Patentansprüche

1. Kunststoff-Implantat zur Anwendung in der Medizin oder Veterinärmedizin, bestehend aus einem Strang eines im Wesentlichen nicht quellfähigen biologisch verträglichen Kunststoffmaterial in Form eines kompakten Zylinders, dahingehend, dass es über die gesamte Länge einen einheitlichen kreisförmigen Querschnitt aufweist und das Kunststoffmaterial eine Shore A-Härte von 0-20 aufweist und das Kunststoff-Implantat aus einem Silikonkautschuk besteht, **dadurch gekennzeichnet,**
**dass** der kompakte Zylinder einen Querschnitt von 0,6-1,8 mm aufweist.

2. Implantat nach Anspruch 1, dahingehend, dass es eine glatte Oberfläche aufweist.

3. Implantat nach Anspruch 1 oder 2, dahingehend, dass es eine Oberflächenbeschichtung zur Verbesserung der Gleitfähigkeit, zur Steigerung der Gewebeverträglichkeit und/oder zur Verhinderung von bakterieller Besiedlung trägt.

4. Implantat nach einem der Ansprüche 1-3, dahingehend, dass es im Inneren mit einem Farbstoff, einem Kontrastmittel, einem pharmakologischen Wirkstoff und/oder einem biologischen Signalstoff beladen ist.

5. Implantat nach einem der Ansprüche 1-4, dahingehend, dass es im Inneren einen oder mehrere kontrastgebende Körper, z.B. einen Faden, Streifen, Partikel und/oder Mikrosphären enthält.

6. Implantat nach einem der Ansprüche 1-5, dahingehend, dass es in sterilisierter und für medizinische Zwecke abgepackter Form vorliegt.

7. Implantat nach einem der Ansprüche 1-6 in Kombination mit einer oder mehreren Einführhilfen.

8. Implantat nach Anspruch 7, dahingehend, dass die Einführhilfe eine nicht schneidende Trokarnadel, gegebenenfalls zusammen mit einer äußeren Trokarhülse, umfasst.

9. Implantat nach Anspruch 7 oder 8, dahingehend dass das Implantat mit einer Trokarnadel verbunden ist.

10. Implantat nach Anspruch 8 oder 9, dahingehend dass die Trokarnadel und gegebenenfalls die Trokarhülse aus chirurgischem Stahl und/oder Kunststoff bestehen, wobei die Oberflächen gegebenenfalls mit einem Gleitmittel beschichtet sind oder eine gleitfähige Oberflächenschicht aufweisen.

11. Implantat nach Anspruch 8-10, dahingehend dass die Trokarnadel aus einem Hartkunststoffmaterial, z.B. einem gehärteten Silikonharz, besteht und fest, insbesondere atraumatisch, mit dem Kunststoff-Implantat verbunden ist.

## Claims

1. Plastics implant for use in the field of medicine or veterinary medicine, consisting of a strand of a substantially non-swellable, biologically compatible plastics material in the form of a compact cylinder, such that said implant has a uniform circular cross section over the entire length thereof, and the plastics material has a Shore A hardness of 0-20 and the plastics implant consists of a silicone rubber, **characterised in that** the compact cylinder has a cross section of 0.6-1.8 mm.

2. Implant according to claim 1, such that said implant has a smooth surface.

3. Implant according to either claim 1 or claim 2, such that said implant has a surface coating for improving slidability, increasing tissue compatibility and/or preventing bacterial colonisation.

4. Implant according to any of claims 1-3, such that said implant is loaded in the interior thereof with a dye, a contrast agent, a pharmacological active substance and/or a biological signalling substance.

5. Implant according to any of claims 1-4, such that said implant contains one or more contrast bodies in the interior thereof, for example a thread, strips, particles and/or microspheres.

6. Implant according to any of claims 1-5, such that said implant is in a form which is sterilised and packaged for medical purposes.

7. Implant according to any of claims 1-6 in combination with one or more insertion aids.

8. Implant according to claim 7, such that the insertion aid comprises a non-cutting trocar needle, optionally together with an outer trocar sleeve.

9. Implant according to either claim 7 or claim 8, such that the implant is connected to a trocar needle.

10. Implant according to either claim 8 or claim 9, such that the trocar needle and optionally the trocar sleeve consist of surgical steel and/or plastics material, the surfaces optionally being coated with a lubricant or having a slidable surface layer.

11. Implant according to claims 8-10, such that the trocar needle consists of a hard plastics material, for example a cured silicone resin, and is rigidly, in particular atraumatically, connected to the plastics implant.

## Revendications

1. Implant en matière plastique destiné à être utilisé en médecine ou en médecine vétérinaire, constitué d'un brin d'une matière plastique biocompatible essentiellement non gonflable sous la forme d'un cylindre compact, de sorte qu'il présente une section transversale circulaire uniforme sur toute sa longueur et que la matière plastique présente une dureté Shore A de 0 à 20 et l'implant en matière plastique est constitué d'un caoutchouc de silicone, **caractérisé en ce que** le cylindre compact présente une section transversale de 0,6 à 1,8 mm.

2. Implant selon la revendication 1, de sorte qu'il présente une surface lisse.

3. Implant selon la revendication 1 ou 2, de sorte qu'il possède un revêtement de surface pour améliorer la capacité de glissement, pour augmenter la compatibilité tissulaire et/ou pour empêcher la colonisation bactérienne.

4. Implant selon l'une des revendications 1 à 3, de sorte qu'il est chargé à l'intérieur d'un colorant, d'un agent de contraste, d'une substance active pharmacologique et/ou d'une substance de signal biologique.

5. Implant selon l'une des revendications 1 à 4, de sorte qu'il contient à l'intérieur un ou plusieurs corps contrastants, par exemple un fil, une bande, des particules et/ou des microsphères.

6. Implant selon l'une des revendications 1 à 5, de sorte qu'il est sous forme stérilisée et emballée à des fins médicales.

7. Implant selon l'une des revendications 1 à 6, en combinaison avec une ou plusieurs aides à l'insertion.

8. Implant selon la revendication 7, de sorte que l'aide à l'insertion comprend une aiguille de trocart non coupante, éventuellement accompagnée d'une douille de trocart extérieure.

9. Implant selon la revendication 7 ou 8, de sorte que l'implant est relié à une aiguille de trocart.

10. Implant selon la revendication 8 ou 9, de sorte que l'aiguille de trocart et éventuellement la douille de trocart sont en acier chirurgical et/ou en matière plastique, les surfaces étant éventuellement revêtues d'un lubrifiant ou présentant une couche superficielle glissante.

11. Implant selon une des revendications 8 à 10, de sorte que l'aiguille de trocart est constituée d'une matière plastique dure, par exemple une résine de silicone durcie, et est fermement, en particulier de manière atraumatique, reliée à l'implant en matière plastique.
